# EUROPÄISCHE PATENTSCHRIFT

(11) **EP 0 832 639 B1**
(45) Veröffentlichungstag und Bekanntmachung des Hinweises auf die Patenterteilung: **28.01.2004**
(21) Anmeldenummer: 97115058.6
(22) Anmeldetag: 30.08.1997
(51) Int. Cl.: A61K 7/11, A61K 7/00

(54) **Verwendung einer Amin enthaltenden Aerosolzusammensetzung**
Use of an amine-containing aerosol composition
Utilisation de composition aérosol contenant une amine

(30) Priorität: 12.09.1996 DE 19637045
(43) Veröffentlichungstag der Anmeldung: 01.04.1998
(73) Patentinhaber: KPSS-Kao Professional Salon Services GmbH, 64297 Darmstadt (DE)
(72) Erfinder: Bünning, Einhard, 64342 Seeheim (DE); Pesch, Ferdinand, Dr., 64625 Bensheim (DE)

(56) Entgegenhaltungen:
- EP-A- 0 274 086
- EP-A- 0 370 338
- EP-A- 0 379 338
- DE-A- 4 422 593
- US-A- 5 304 368
- US-A- 5 374 420

## Beschreibung

Die vorliegende Erfindung betrifft die Verwerdung einer Aerosolzusammensetzung auf Grundlage mindestens eines Lösungsmittels, insbesondere niederer Alkohole, und mindestens eines Treibmittels, insbesondere einen Haarspray.

Aerosolzusammensetzungen werden entweder in Weißblechdosen, die üblicherweise eine Schweißnaht aufweisen, oder Aluminiummonoblockdosen, die mit einem Innenschutzlack versehen sind, abgefüllt. Dabei kommt es immer wieder zu punktuellen Korrosionserscheinungen, insbesondere im Bereich der Schweißnaht oder des Bördelrandes.

Obwohl bereits viele Vorschläge zur Lösung dieses Problems durch Zusatz der verschiedensten Korrosionsinhibitoren unterbreitet wurden, besteht nach wie vor ein Bedürfnis, nicht korrodierende Aerosolzusammensetzungen zur Abpackung in Weißblech- oder Aluminium-Aerosoldosen zu entwickeln.

Es wurde nunmehr gefunden, daß durch den Einsatz geringster Mengen mindestens eines Amins aus der Gruppe 2-Amino-2-methyl-1-propanol, 2-Amino-2-methyl-1,3-propandiol und/oder Triisopropanolamin in konventionellen Aerosolzusammensetzungen eine korrosionsverhindernde Wirkung erreicht werden kann.

Als Mindestmenge sind dabei schon 0,001 Gew.-%, berechnet auf die Gesamtzusammensetzung, wirksam; ein Bereich von 0,01 bis 0,1 Gew.-% ist besonders bevorzugt.

Eine bevorzugt verwendete Aerosolzusammensetzung sind Haarsprays auf alkoholischer bzw. wäßrig-alkoholischer Basis.

Als Korrosionsschutzmittel für solche Haarsprays sind beispielsweise bereits organische Phosphate bzw. quaternäre Ammoniumverbindungen vorgeschlagen worden (vgl. die Monographie von K. Schrader, "Grundlagen und Rezepturen der Kosmetika", 2. Aufl. (1989), S. 885), doch haben sich diese nicht bewährt.

Aus den Patent- oder Offenlegungsschriften US 5,374,420, EP 370 338 A, EP 274 086 A, DE 44 22 593 A und US 5,304,368 ist die Verwendung von Amin-Substanzen in Aerosolzusammensetzungen auf dem Gebiet der Haarpflege zur Neutralisierung bekannt, jedoch ist in keiner dieser Druckschriften der korrosionsschützende Effekt genannt.

Auch der Einsatz von Natriumbenzoat in Aerosolzusammensetzungen ist für diesen Zweck bereits vorgeschlagen worden, jedoch ist dessen Wirksamkeit vom pH-Wert abhängig.

Aus der EP-A 382 061 ist ein Verfahren zur Inhibierung der Korrosion in Dampf-/Kondenswassersystemen durch Zusatz von Aminodiolen, vorzugsweise im Gemisch mit flüchtigen weiteren Aminen, zu Kesselspeisewasser bekannt.

Da es sich hierbei um andere Materialien und Zusammensetzungen handelt, konnte der Fachmann diesem Stand der Technik keinerlei Anregung für die Lösung der erfindungsgemäßen Aufgabe entnehmen.

Gleiches gilt im Hinblick auf die US-A 3 976 441, die die Verwendung von substituierten Aminoalkylpropandiolen als Rost- und Korrosionsinhibitoren in Motoröl offenbart.

Haarsprays, die im Rahmen der Erfindung besonders geeignet sind, enthalten vorzugsweise 15 bis 70 Gew.-% eines Treibmittels oder Treibmittelgemisches, z. B. n-Butan, Isobutan, Propan, n-Pentan (das auch als Lösungsmittelbestandteil betrachtet werden kann), und/oder Dimethylether.

Auch die Verwendung komprimierter Treibmittel wie Luft oder Kohlendioxid ist möglich, wobei sich die Mengenanteile dann durch den erforderlichen Druck (CO₂: 4 - 6 bar; Luft bis 12 bar) ergeben.

Als Lösungsmittel finden insbesondere niedere Alkohole wie Ethanol, n-Propanol und Isopropylalkohol in einer bevorzugten Menge von 20 bis 70, insbesondere 30 bis 50 Gew.-%, berechnet auf die Gesamtzusammensetzung, Verwendung, wobei, zur Herstellung sogenannter "Low VOC-Sprays", der Alkoholanteil bis zu etwa 75 Gew.-%, vorzugsweise 15 bis 50 Gew.-%, durch Wasser ersetzt sein kann.

Die verwendeten Haarsprays enthalten mindestens 0,5 bis 10 Gew.-% eines Filmbildners, vorzugsweise eines nichtionischen und/oder kationischen Polymeren.

Verwendbare nichtionische Polymere sind Vinylpyrrolidon/Vinylacetat/Vinylpropionat-Copolymere wie "Luviskol VAP 343®", Vinylpyrrolidon/(Meth)Acrylsäureester-Copolymere sowie Chitosan-Derivate.

Ihr Anteil in den verwendeten Haarbehandlungsmitteln liegt zwischen etwa 0,25 und etwa 10,0, vorzugsweise 0,5 und 7,5, insbesondere etwa 1 bis 2,5 und 5 Gew.-%, bezogen auf die Gesamtzusammensetzung des Mittels.

Eine große Variationsbreite besteht auch bei den kationischen Polymeren, die in einer Menge zwischen etwa 0,1 und etwa 7,5 bis 10 Gew.-%, vorzugsweise 0,25 bis 5, insbesondere etwa 0,50 bis 7,5 Gew.-%, je nach Charakter des Polymeren, enthalten sein können, auch im Gemisch mit den nichtionischen Polymeren.

Kationische Polymere sind neben den altbekannten quaternären Cellulosederivaten des Typs "Polymer JR" insbesondere quaternisierte Homo- und Copolymere des Dimethyldiallylammoniumchlorids, wie sie unter dem Handelsnamen "Merquat" im Handel sind, quaternäre Vinylpyrrolidon-Copolymere, insbesondere mit Dialkylaminoalkyl(meth)acrylaten, wie sie unter dem Namen "Gafquat" bekannt sind, Copolymerisate aus Vinylpyrrolidon und Vinylimidazoliniummethochlorid, die unter dem Handelsnamen "Luviquat" angeboten werden, Polyamino-Polyamid-Derivate, beispielsweise Copolymere von Adipinsäure-Dimethylaminohydroxypropyldiethylentriamin, wie sie unter dem Namen "Cartaretine F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" im Handel sind.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden. Der bevorzugte Anteil eines solchen kationischen Polymeren liegt bei 0,1 bis 5, vorzugsweise 0,25 bis 2,5, insbesondere 0,5 bis 1,5 Gew.-% des Mittels.

Geeignet sind auch die in der EP-A 640 643 beschriebenen kationisch derivatisierten Organopolysiloxane, insbesondere quaternierte Aminoalkyldimethylpolysiloxan/Polyethyloxazolin-Copolymeren.

Die Verwendung anionischer und/oder amphoterer Polymerer, gegebenenfalls im Gemisch mit nichtionischen und/oder kationischen Polymeren, ist ebenfalls möglich.

Die Verwendung der genannten Amine als Neutralisationsmittel für diese anionischen bzw. amphoteren Polymeren ist natürlich bekannt, wobei erfindungsgemäß dann entsprechend höhere Mengen Amin, zusätzlich und in Abhängigkeit vom Anteil der zu neutralisierenden Carboxyl- oder Sulfonsäuregruppen, zur Korrosionsverhinderung zum Einsatz kommen.

Dementsprechend verstehen sich also die Aminanteile, die zur Korrosionsinhibierung verwendet werden, im Falle des Vorliegens carboxyl- und/oder sulfogruppenhaltiger Filmbildner zusätzlich zu dem für die Neutralisation ohnehin erforderlichen Gehalt an Aminen.

Eine weitere Gruppe von Aerosolzusammensetzungen, bei denen die erfindungsgemäße Korrosionsstabilisierung Anwendung finden kann, sind Schaumaerosole auf wäßriger Basis.

Derartige Produkte sind, ebenso wie Haarsprays, aus dem Stand der Technik an sich ebenfalls bekannt.

Im folgenden wird das Verhalten der erfindungsgemäß verwendeten Zusammensetzung im Vergleich zu konventionellen Zusammensetzungen bei sechsmonatiger Lagerung bei 40 °C in Weißblechdosen, zusammengesetzt entsprechend der Euronorm EN 10203, beschrieben.

### Beispiel 1

| | |
|---|---|
| Ethanol | 23,76 (Gew.-%) |
| Isopropylalkohol | 10,00 |
| Phenyl Dimethicone (200 mm² s⁻¹) | 0,20 |
| PVP/VA Copolymer (50%ig) | 10,00 |
| Triisopropanolamin | 0,04 |
| n-Butan | 26,00 |
| Dimethylether | 30,00 |

### Beispiel 1A

Identisch mit Beispiel 1; jedoch kein Triisopropanolamin, dafür 23,80 Gew.-% Ethanol.

### Beispiel 2

| | |
|---|---|
| Ethanol | 38,78 (Gew.-%) |
| Isopropylmyristat | 0,20 |
| PVP/VA Copolymer (50%ig) | 8,00 |
| Polyquaternium-11 (50%ig) | 1,00 |
| 2-Amino-2-methyl-1-propanol (AMP) | 0,02 |
| n-Pentan | 24,00 |
| Dimethylether | 28,00 |

### Beispiel 2A

Identisch mit Beispiel 2; jedoch kein AMP, dafür 38,80 Gew.-% Ethanol.

### Beispiel 3

| | |
|---|---|
| Ethanol | 26,26 (Gew.-%) |
| Wasser | 5,00 |
| Isopropylmyristat | 0,20 |
| Dow Corning® Cosmetic Wax 2501 | 0,50 |
| PVP/VA Copolymer (50%ig) | 12,00 |
| 2-Amino-2-methyl-1,3-propandiol | 0,04 |
| (AMPD) | |
| n-Pentan | 24,00 |
| Dimethylether | 32,00 |

### Beispiel 3A

Identisch mit Beispiel 3; jedoch kein AMPD, dafür 26,30 Gew.-% Ethanol.

Jeweils 10 Weißblechdosen wurden mit Zusammensetzungen nach den vorangegangenen Beispielen gefüllt.

Nach sechsmonatiger Lagerung bei 40°C wurden die Dosen geöffnet und auf etwaige Korrosionspunkte untersucht.

| Ergebnis | | | | | | |
|---|---|---|---|---|---|---|
| **Zusammensetzung nach Beispiel** | **1** | **1A** | **2** | **2A** | **3** | **3a** |
| Zahl der Dosen mit Korrosion, insbesondere an der Schweißnaht und am Bördelrand | 0 | 8 | 0 | 8 | 0 | 10 |

Dieses Ergebnis zeigt den markanten Unterschied im Korrosionsverhalten zwischen den erfindungsgemäßen und den nicht stabilisierten Zusammensetzungen.

Weitere Beispiele für erfindungsgemäß zusammengesetzte Produkte:

### Beispiel 4

| Haarspray | |
|---|---|
| Ethanol | 23,78 (Gew.-%) |
| Isopropylalkohol | 10,00 |
| Phenyl Dimethicone | 0,20 |
| PVP/VA Copolymer (50%ig) | 10,00 |
| AMP | 0,01 |
| AMPD | 0,01 |
| Parfum | 0,25 |
| n-Butan | 26,00 |
| Dimethylether | 30,00 |

### Beispiel 5

| Haarspray | |
|---|---|
| Ethanol | 23,25 (Gew.-%) |
| Isopropylalkohol | 10,00 |
| Phenyl Dimethicone | 0,20 |
| Polysilicone-9 (kationisches Aminoalkyldimethylpolysiloxan/Polyethyloxazolin-Copolymer nach EP-A 640 643) | 0,50 |
| PVP/VA/Vinyl Propionate Copolymer (50%ig) | 10,00 |
| AMP | 0,02 |
| Triisopropanolamin | 0,03 |
| Parfum | 0,30 |
| n-Pentan | 26,00 |
| Dimethylether | 30,00 |

### Beispiel 6

| Haarspray | |
|---|---|
| Ethanol | 35,00 (Gew.-%) |
| PVP/VA Copolymer (50%ig) | 10,00 |
| Parfum | 0,30 |
| AMP | 0,01 |
| Dimethylether | 40,00 |
| Difluorethan (152 A) | @ 100,00 |

### Beispiel 7

| Haarspray | |
|---|---|
| Ethanol | 51,50 (Gew.-%) |
| PVP/VA Copolymer (50%ig) | 3,00 |
| Vinyl Caprolactam/PVP/Dimethylaminoethyl Methacrylate Copolymer | 7,20 |
| Natriumstearoyllactylat | 1,00 |
| Parfum | 0,20 |
| AMPD | 0,05 |
| Dimethylether | 25,00 |
| Wasser | @ 100,00 |

### Beispiel 8

| Low VOC-Haarspray | |
|---|---|
| PVP/VA Copolymer (50%ig) | 10,00 (Gew.-%) |
| Ethanol | 15,00 |
| AMP | 0,05 |
| Dimethylether | 35,00 |
| Parfum | 0,30 |
| Wasser | @ 100,00 |

### Beispiel 9

| Schaumspray | |
|---|---|
| PVP/VA Copolymer (50%ig) | 4,00 (Gew.-%) |
| Ethanol | 8,00 |
| Ceteareth-25 | 0,20 |
| Hydroxyethyl Cetyldimonium | 0,50 |
| Phosphate | |
| Cetrimoniumchlorid | 0,50 |
| Parfum | 0,30 |
| Propan/Butan | 7,00 |
| AMP | 0,03 |
| AMPD | 0,03 |
| Wasser | @ 100,00 |

Nach sechsmonatiger Lagerung dieser Zusammensetzung in Weißblechdosen bei 40 °C wurden keinerlei Korrosionserscheinungen beobachtet.

## Patentansprüche

1. Verwendung mindestens eines Amins aus der Gruppe 2-Amino-2-methyl-1-propanol, 2-Amino-2-methyl-1,3-propandiol und/oder Triisopropanolamin in Aerosolzusammensetzungen auf Basis mindestens eines Lösungs- und mindestens eines Treibmittels und gegebenenfalls Wasser in einer Menge von mindestens 0,001 Gew.-%, bezogen auf die Gesamtzusammensetzung, zur Korrosionsverhinderung.

2. Verwendung mindestens eines Amins in Aerosolzusammensetzungen nach Anspruch 1, in einer Menge von 0,01 bis 0,1 Gew.-%, berechnet auf die Gesamtzusammensetzung.

3. Verwendung mindestens eines Amins in Aerosolzusammensetzungen nach Anspruch 1 oder 2, enthaltend 0,5 bis 10 Gew.-% mindestens eines Filmbildners, 20 bis 70 Gew.-% mindestens eines Alkohols aus der Gruppe Ethanol, n-Propanol und/oder Isopropylalkohol, mindestens ein Treibmittel aus der Gruppe Propan, Butan, Isobutan, n-Pentan, Dimethylether, Luft und/oder Kohlendioxid, gegebenenfalls Wasser, jeweils berechnet auf die Gesamtzusammensetzung.

4. Verwendung eines Amins in Aerosolzusammensetzungen nach Anspruch 3, enthaltend als Filmbildner mindestens ein nichtionisches und/oder kationisches Polymer.

## Claims

1. Use of at least one amine compound selected from the group 2-amino-2-methyl-1-propanol, 2-amino-2-methyl-1,3-propanediol and/or triisopropanol-amine in an amount of at least 0.001 % by weight, calculated to the total composition, in aerosol compositions for protection against corrosion based on at least one solvent and at least one propellant agent and optionally water.

2. Use of at least one amine compound in an amount of 0.01 to 0.1 % by weight, calculated on the total composition in aerosol compositions according to claim 1.

3. Use of at least one amine compound in aerosol compositions according to claim 1 or 2, containing 0.5 to 10% by weight of at least one film-forming agent, 20 to 70% by weight of at least one alcohol from the group ethanol, n-propanol and/or isopropyl alcohol, at least one propellant selected from the group propane, butane, isobutane, n-pentane, dimethyl ether, air and/or carbon dioxide, optionally water, calculated to the total composition.

4. Use of one amine compound in aerosol composition according to claim 3, containing as the film-forming agent at least one nonionic and/or cationic polymer.

## Revendications

1. Utilisation d'au moins une amine du groupe constitué du 2-amino-2-méthyl-1-propanol, du 2-amino-2-méthyl-1,3-propanediol et de la triisopropanolamine dans des compositions aérosol à base d'au moins un solvant et d'au moins un agent propulseur et éventuellement d'eau, en quantité d'au moins 0,001 % en poids par rapport à la composition totale, pour la protection contre la corrosion.

2. Utilisation d'au moins une amine dans des compositions aérosol selon la revendication 1, en quantité d'au moins 0,01 à 0,1 % en poids par rapport à la composition totale.

3. Utilisation d'au moins une amine dans des compositions aérosol selon la revendication 1 ou 2, qui contiennent de 0,5 à 10 % en poids d'au moins un agent de formation de film, de 20 à 70 % en poids d'au moins un alcool du groupe constitué de l'éthanol, du n-propanol et de l'alcool propylique, au moins un agent propulseur du groupe constitué du propane, du butane, de l'isobutane, du n-pentane, de l'éther de diméthyle, de l'air et du dioxyde de carbone, avec éventuellement de l'eau, chaque fois par rapport à la composition totale.

4. Utilisation d'une amine dans des compositions aérosol selon la revendication 3, qui contient comme agent de formation de film au moins un polymère non ionique et/ou cationique.
